# EUROPEAN PATENT SPECIFICATION

(11) **EP 3 238 698 B1**
(45) Date of publication and mention of the grant of the patent: **05.08.2020**
(21) Application number: 15873261.0
(22) Date of filing: 25.12.2015
(51) Int. Cl.: A61K 8/44, A61K 8/34, A61K 8/60, A61Q 19/00

(54) **COSMETIC COMPOSITION CONTAINING WATER-SOLUBLE MOISTURIZING COMPONENT AND ACYL BASIC AMINO ACID DERIVATIVE**
KOSMETISCHE ZUSAMMENSETZUNG MIT WASSERLÖSLICHER FEUCHTIGKEITSSPENDENDER KOMPONENTE UND BASISCHEM ACYLAMINOSÄUREDERIVAT
COMPOSITION COSMÉTIQUE CONTENANT UN COMPOSANT HYDRATANT SOLUBLE DANS L'EAU ET UN DÉRIVÉ D'ACIDE AMINÉ BASIQUE ACYLÉ

(30) Priority: 25.12.2014 JP 2014262706
(43) Date of publication of application: 01.11.2017
(73) Proprietor: AJINOMOTO CO., INC., Chuo-ku Tokyo 104-8315 (JP)
(72) Inventor: KOBAYASHI, Shun, Kawasaki-shi Kanagawa 210-8681 (JP); OSHIMURA, Eiko, Kawasaki-shi Kanagawa 210-8681 (JP)
(74) Representative: Mewburn Ellis LLP
(86) International application number: PCT/JP2015/086210
(87) International publication number: WO 2016/104693

(56) References cited:
- EP-A1- 1 473 027
- EP-A1- 2 813 487
- WO-A1-2013/118896
- DE-U1- 20 319 986
- JP-A- 2004 323 505
- MASAHIRO SUZUKI ET AL.: 'Novel dumbbell-form low- molecular-weight gelators based on L-lysine: their hydrogelation and organogelation properties' NEW JOURNAL OF CHEMISTRY vol. 29, no. 11, 01 January 2005, pages 1439 - 1444, XP055346030 DOI: 10.1039/B511158G
- Anonymous: "DMSO Creme Dimethylsulfoxid 99,9% Reinheit - 50ml - in einer hochwertigen Basicreme nach DAC Deutschem Apotheken Codex: Amazon.de: Beauty", , 26 July 2019 (2019-07-26), XP055609069, Retrieved from the Internet: URL:https://www.amazon.de/DMSO-Creme-Dimet hylsulfoxid-Reinheit-hochwertigen/dp/B07NP DK8Z6/ref=asc_df_B07NPDK8Z6/?tag=googshopd e-21&linkCode=df0&hvadid=309008166883&hvpo s=1o2&hvnetw=g&hvrand=11682487062305862956 &hvpone=&hvptwo=&hvqmt=&hvdev=c&hvdvcmdl=& hvlocint=&hvlocphy=1004234&hvtargid=pla-70 3063365315 [retrieved on 2019-07-26]
- Anonymous: "Dimethyl Sulfoxide - High Purity Solvents | Sigma-Aldrich", , 26 July 2019 (2019-07-26), XP055609093, Retrieved from the Internet: URL:https://www.sigmaaldrich.com/chemistry /solvents/dimethyl-sulfoxide-center.html [retrieved on 2019-07-26]

## Description

### [Technical Field]

The present invention relates to a composition containing (A): an acyl basic amino acid derivative, (B): a water-soluble moisturizing component, and (C): water, which is used as, for example, cosmetic. The definition of the invention in the further context of the following description and the examples is strictly limited to the definition of the components (A), (B) and (C) and their relative amounts as provided in the appended claims.

### [Background Art]

N^{ε}-lauroyllysine is used for cosmetics and the like since it shows properties of good slipperiness and good spreadability, less irritation to the skin, good attachability to the skin, reduction of "greasiness" and "stickiness" derived from oil agent and moisturizer and the like (patent documents 1 - 4 etc.). However, since N^{ε}-lauroyllysine is poorly soluble in water and oil, it has a problem of limited use for a solid (powder), and unavailability as a transparent aqueous cosmetic. In addition, since N^{ε}-lauroyllysine has high water-repellency, affinity to water is poor, which in turn problematically causes difficulty in being stably blended in an aqueous cosmetic. Furthermore, since N^{ε}-lauroyllysine coagulates in the obtained aqueous cosmetic, the cosmetic problematically loses smoothness.

It has been reported that a compound represented by the following formula: 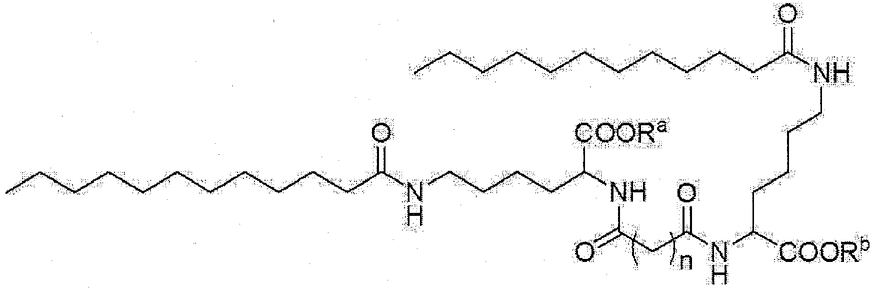 wherein R^{a} and R^{b} are each a hydrogen atom or an alkyl group, and n is an integer of 0 to 12, or a salt thereof (hereinafter to be also referred to as "lauroyl amino acid derivative") is useful for gelation or solidifying water and a liquid organic medium (patent document 5, non-patent document 1 and non-patent document 2 etc.).

However, a composition containing a lauroylamino acid derivative, water-soluble moisturizing component and water, and a cosmetic containing the composition have not been reported heretofore.

### [Document List]

### [Patent documents]

patent document 1: WO 01/014317
patent document 2: JP-A-61-137812
patent document 3: JP-A-60-67406
patent document 4: JP-A-3-74312
patent document 5: JP-A-2004-323505

### [non-patent document]

non-patent document 1: Org. Biomol. Chem., 2003, 1, 4124-4131
non-patent document 2: New J. Chem., 2005, 29, 1439-1444

### [SUMMARY OF THE INVENTION]

### [Problems to be Solved by the Invention]

An object of the present invention is to provide a stable composition which is superior in affinity and adhesion to the skin and hair, easily provides effects as a cosmetic, and is also superior in the sustainability of the effect.

### [Means of Solving the Problems]

The present inventors have conducted intensive studies in an attempt to achieve the above-mentioned object and found that a composition containing component (A): a compound represented by the following formula (1) (hereinafter sometimes to be also referred to as "compound (1)") or a salt thereof, component (B): a water-soluble moisturizing component and (C): water has moist but does not become sticky, is superior in affinity and adhesion to the skin and hair, easily provides effects as a cosmetic, is also superior in the sustainability of the effect, is prepared easily and is superior in stability, which resulted in the completion of the present invention.

Therefore, the present invention provides the following.
[1] A composition comprising component (A): a compound represented by the formula (1) 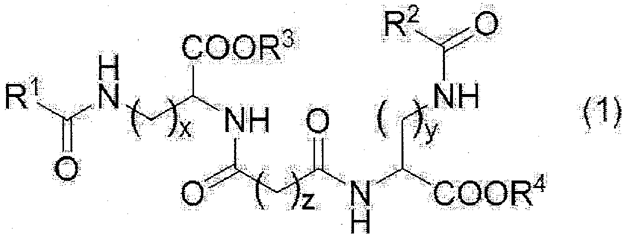 wherein
   R¹ and R² are each independently an alkyl group having 5 - 21 carbon atoms or an alkenyl group having 5 - 21 carbon atoms,
   R³ and R⁴ are each independently a hydrogen atom, an alkyl group having 1 - 22 carbon atoms or an alkenyl group having 2 - 22 carbon atoms, z is an integer of not less than 0,
   x and y are each independently an integer of 2 - 4, or a salt thereof,
   component (B): a water-soluble moisturizing component; and
   component (C): water.
[2] The composition of [1], wherein component (A) is a compound of the aforementioned formula (1) wherein z is an integer of 0 - 10, or a salt thereof.
[3] The composition of [1] or [2], wherein component (A) is a compound of the aforementioned formula (1) wherein z is 7 or 8, or a salt thereof.
[4] The composition of any of [1] - [3], wherein component (A) is a compound of the aforementioned formula (1) wherein x and y are each 4, or a salt thereof.
[5] The composition of any of [1] - [4], wherein component (A) is a compound of the aforementioned formula (1) wherein R¹ and R² are each independently a straight-chain alkyl group having 5 - 15 carbon atoms, or a salt thereof.
[6] The composition of any of [1] - [5], wherein component (A) is a compound of the aforementioned formula (1) wherein R³ and R⁴ are each a hydrogen atom, or a salt thereof.
[7] The composition of any of [1] - [5], wherein component (A) is a compound of the aforementioned formula (1) wherein R¹ and R² are each independently a straight-chain alkyl group having 5 - 15 carbon atoms, R³ and R⁴ are each a hydrogen atom, z is an integer of 0 - 10, and x and y are each 4, or a salt thereof.
[8] The composition of any of [1] - [5], wherein component (A) is a compound of the aforementioned formula (1) wherein R¹ and R² are each a straight-chain alkyl group having 5 - 15 carbon atoms, R³ and R⁴ are each a hydrogen atom, z is 7 or 8, and x and y are each 4, or a salt thereof.
[9] The composition of any of [1] - [8], wherein component (A) is a compound selected from bis(N^{ε}-lauroyl-L-lysine)sebacoyl amide or a salt thereof, and bis(N^{ε}-octanoyl-L-lysine)sebacoyl amide, or a salt thereof.
[10] The composition of any of [1] - [9], wherein component (B) is at least one kind of water-soluble moisturizing component selected from the group consisting of polyols and saccharides.
[11] The composition of any of [1] - [10], further comprising component (D): a nonionic surfactant.
[12] The composition of [11], wherein component (D) is at least one kind of nonionic surfactant selected from the group consisting of polyoxyalkylene ethers, polyhydric alcohol fatty acid esters, polyoxyalkylene esters, polyoxyethylene dimer diol ether, polyoxyethylene glyceryl ether fatty acid ester and ether modified dimethicone.
[13] The composition of any of [1] - [10], further comprising component (E): an oil agent and/or component (F): a water-soluble polymer.
[14] The composition of [11] or [12], further comprising component (E): an oil agent and/or component (F): a water-soluble polymer.
[15] The composition of any of [1] - [14], wherein component (A) is contained in a proportion of 0.005 - 20 wt% relative to the total amount of the composition.
[16] A cosmetic comprising the composition of any of [1] - [15].

### [Effect of the Invention]

According to the present invention, a stable composition, which is superior in the affinity and adhesion to the skin and hair, easily provides effects as a cosmetic such as moisturizing effect (conferring moist feeling) and conditioning effect (improvement of the surface condition of the skin and hair) and the like, and is also superior in the sustainability of the effect, can be provided.

### [Brief Description of the Drawings]

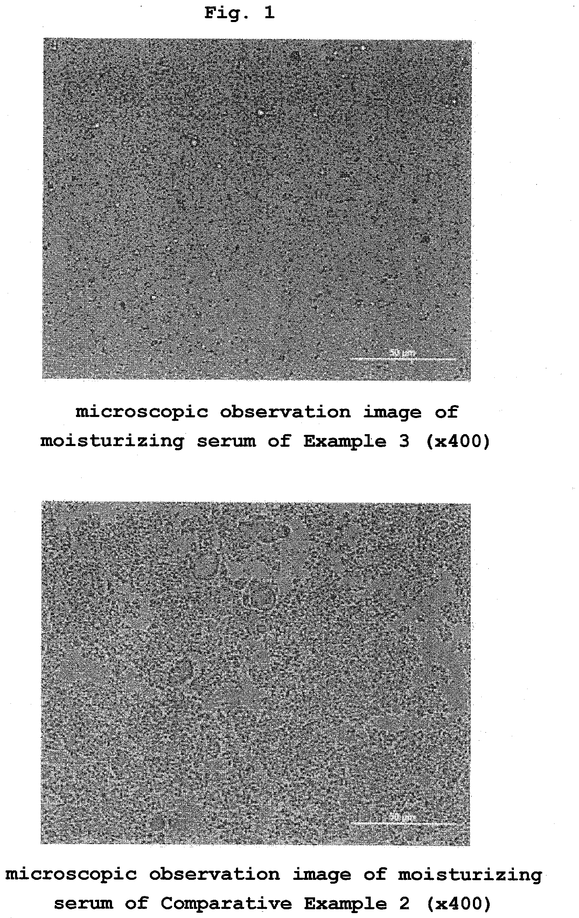

Fig. 1 shows microscopic observation images of the moisturizing serum of Example 3 and the moisturizing serum of Comparative Example 2.

### [Description of Embodiments]

The composition of the present invention is characterized in that it is a composition containing component (A): a compound represented by the formula (1) 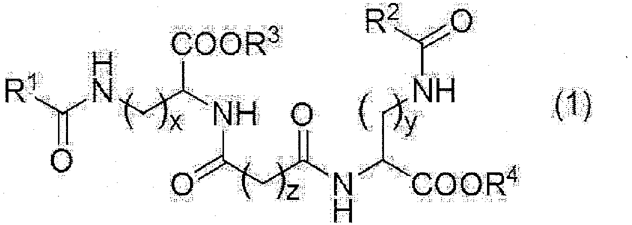 wherein
R¹ and R² are each independently an alkyl group having 5 - 21 carbon atoms or an alkenyl group having 5 - 21 carbon atoms,
R³ and R⁴ are each independently a hydrogen atom, an alkyl group having 1 - 22 carbon atoms or an alkenyl group having 2 - 22 carbon atoms,
z is an integer of not less than 0,
x and y are each independently an integer of 2 - 4, or a salt thereof,
component (B): a water-soluble moisturizing component, and
component (C): water.

In addition, the composition of the present invention is characterized in that it is a composition further containing component (D): a nonionic surfactant, in addition to component (A), component (B) and component (C).

Furthermore, the composition of the present invention is characterized in that it is a composition further containing component (E): an oil agent and/or component (F): a water-soluble polymer, in addition to component (A), component (B), component (C) and component (D).

The embodiment of the present invention is described in detail in the following.

### 1. Component (A): a compound represented by the formula (1) (compound (1)) or a salt thereof

R¹ and R² are each independently an alkyl group having 5 - 21 carbon atoms or an alkenyl group having 5 - 21 carbon atoms.

The alkyl group having 5 - 21 carbon atoms means a straight-chain or branched-chain alkyl group having 5 - 21 carbon atoms. Specific examples thereof include pentyl group, isopentyl group, neopentyl group, a hexyl group, isohexyl group, neohexyl group, heptyl group, isoheptyl group, neoheptyl group, octyl group, isooctyl group, nonyl group, isononyl group, decyl group, isodecyl group, undecyl group, dodecyl group, tridecyl group, tetradecyl group, pentadecyl group, hexadecyl group, heptadecyl group, octadecyl group, nonadecyl group, icosyl group and the like.

The alkenyl group having 5 - 21 carbon atoms means a straight-chain or branched-chain alkenyl group having 5 - 21 carbon atoms. Specific examples thereof include pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group and the like.

An alkyl group having 5 - 15 carbon atoms means a straight-chain or branched-chain alkyl group having 5 - 15 carbon atoms. Specific examples thereof include pentyl group, a hexyl group, heptyl group, octyl group, nonyl group, decyl group, undecyl group, dodecyl group, tridecyl group, tetradecyl group, pentadecyl group and the like.

An alkyl group having 7 - 11 carbon atoms means a straight-chain or branched-chain alkyl group having 7 - 11 carbon atoms. Specific examples thereof include heptyl group, octyl group, nonyl group, decyl group, undecyl group and the like.

R¹ and R² are preferably each independently an alkyl group having 5 - 15 carbon atoms, more preferably each independently an alkyl group having 7 - 11 carbon atoms.

Preferably, R¹ and R² are each a straight chain alkyl group. Furthermore, R¹ and R² are preferably the same.

R³ and R⁴ are each independently a hydrogen atom, an alkyl group having 1 - 22 carbon atoms or an alkenyl group having 2 - 22 carbon atoms.

An alkyl group having 1 - 22 carbon atoms means a straight-chain or branched-chain alkyl group having 1 - 22 carbon atoms. Specific examples thereof include methyl group, an ethyl group, a propyl group, an isopropyl group, a butyl group, an isobutyl group, a sec-butyl group, a tert-butyl group, a pentyl group, isopentyl group, neopentyl group, a hexyl group, isohexyl group, neohexyl group, heptyl group, isoheptyl group, neoheptyl group, octyl group, isooctyl group, nonyl group, isononyl group, decyl group, isodecyl group, undecyl group, dodecyl group, tridecyl group, tetradecyl group, pentadecyl group, hexadecyl group, heptadecyl group, octadecyl group, nonadecyl group, icosyl group and the like.

An alkenyl group having 2 - 22 carbon atoms means a straight-chain or branched-chain alkenyl group having 2 - 22 carbon atoms. Specific examples thereof include ethenyl group, 1-propenyl group, 2-propenyl group, 1-butenyl group, 2-butenyl group, 3-butenyl group, pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group and the like.

Preferably, both R³ and R⁴ are hydrogen atoms.

z is an integer of not less than 0.

z is preferably an integer of 0 - 10, more preferably 7 or 8.

x and y are each independently an integer of 2 - 4.

x and y are each preferably 4.

As a compound represented by the formula (1), the following compounds can be preferably recited.

### (compound A)

A compound wherein R¹ and R² are each independently a straight-chain alkyl group having 5 - 15 carbon atoms,
R³ and R⁴ are each a hydrogen atom,
z is an integer of 0 - 10, and
x and y are each 4.

### (compound B)

A compound wherein R¹ and R² are each a straight chain alkyl group having 5 - 15 carbon atoms,
R³ and R⁴ are each a hydrogen atom,
z is 7 or 8, and
x and y are each 4.

### (compound C)

A compound wherein R¹ and R² are each a straight chain alkyl group having 7 - 11 carbon atoms,
R³ and R⁴ are each a hydrogen atom,
z is 7 or 8, and
x and y are each 4.

Specific examples of the compound represented by the formula (1) include
bis(N^{ε}-lauroyl-L-lysine)sebacoyl amide,
bis(N^{ε}-octanoyl-L-lysine)sebacoyl amide, and a salt thereof.

The salt of the compound represented by the formula (1) is not particularly limited. Examples thereof include alkali metal salts such as sodium salt, potassium salt and the like, alkaline earth metal salts such as calcium salt, magnesium salt and the like, inorganic salts such as aluminum salt, salt with zinc and the like, and organic salts such as organic amine salts such as ammonium salt, monoethanolamine salt, diethanolamine salt, triethanolamine salt and the like, basic amino acid salts such as arginine salt, lysine salt and the like, and the like. One kind of these may be used, or two or more kinds selected from the above-mentioned group may be used in a mixture. From the aspects of easy availability, handling property and the like, alkali metal salt, organic amine salt, or basic amino acid salt is preferable, and sodium salt and potassium salt are particularly preferable.

Compound (1) can be produced by a method conventional method (JP-A-2004-323505, Org. Biomol. Chem., 2003, 1, 4124-4131, New J. Chem., 2005, 29, 1439-1444 etc.). For example, as shown in the following formula, of compounds (1), symmetrical compound (1') can be produced by reacting N^{ω}-acyl amino acid (2) and dicarboxylic acid dichloride (3) in an appropriate solvent. 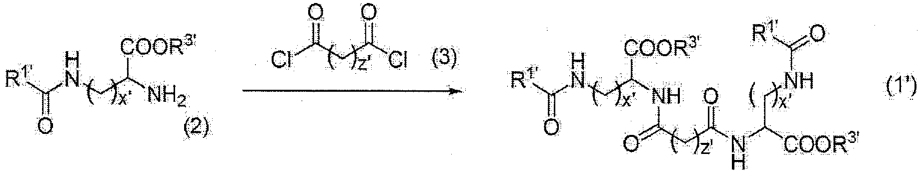 wherein R^{1'} is an alkyl group having 5 - 21 carbon atoms or an alkenyl group having 5 - 21 carbon atoms, R^{3'} is a hydrogen atom, an alkyl group having 1 - 22 carbon atoms or an alkenyl group having 2 - 22 carbon atoms, z' is an integer of not less than 0, and x' is an integer of 2 - 4.

Examples of the N^{ω}-acyl amino acid (2) include N^{ε}-acyl lysine (e.g., N^{ε}-hexanoyl-L-lysine, N^{ε}-octanoyl-L-lysine etc.), N^{δ}-acyl ornithine (e.g., N^{δ}-hexanoyl-L-ornithine etc.), N^{γ}-acyl-α,γ-diaminobutyric acid and the like.

Examples of the dicarboxylic acid dichloride (3) include oxalyl chloride, malonyl chloride, succinyl chloride, glutaryl chloride, adipoyl chloride, pimeloyl chloride, suberoyl chloride, azelaoyl chloride, sebacoyl chloride, dodecanedioyl chloride and the like. The amount of dicarboxylic acid dichloride (3) to be used is generally 0.4 - 0.6 equivalent relative to N^{ω}-acyl amino acid (2).

While the solvent is not particularly limited as long as it is inert to the reaction, examples thereof include ethers such as diethyl ether, tetrahydrofuran and the like.

In addition, of compounds (1), asymmetric compound (1") can be produced as follows. First, N^{ω}-acyl amino acid (2) and dicarboxylic acid monochloride monoester (4) are reacted in an appropriate solvent to give compound (5) (step 1). Then, the primary ester moiety of the obtained compound (5) is hydrolyzed in the presence of a base such as sodium hydroxide, potassium hydroxide and the like, the carboxylic acid moiety is chlorinated with a chlorinating agent such as thionyl chloride and the like, and the compound is reacted with N^{ω}-acyl amino acid (2') which is different from N^{ω}-acyl amino acid (2) used in the aforementioned step 1 (step 2), whereby derivative (1") can be produced. 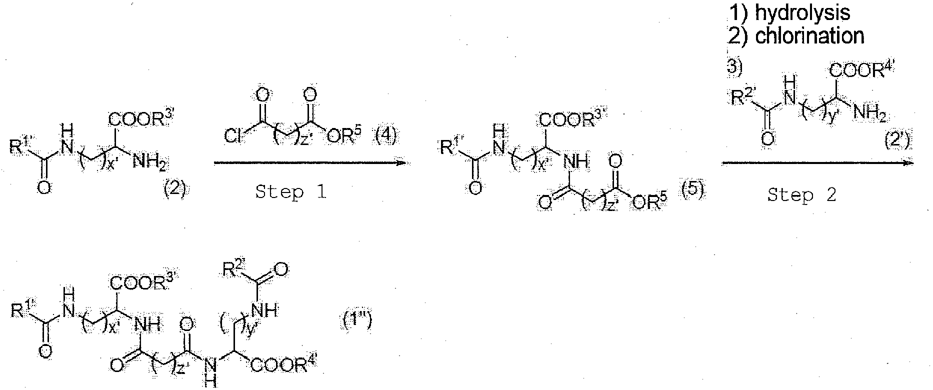 wherein R^{1'}, R^{3'}, z' and x' are as defined above, R^{2'} is an alkyl group having 5 - 21 carbon atoms or an alkenyl group having 5-21 carbon atoms, R^{4'} is a hydrogen atom, an alkyl group having 1 - 22 carbon atoms or an alkenyl group having 2 - 22 carbon atoms, R⁵ is an alkyl group such as a methyl group, an ethyl group and the like, and y' is an integer of 2 - 4.

As Nω-acyl amino acids (2) and (2'), N^{ω}-acyl amino acids similar to those mentioned above can be used.

As dicarboxylic acid monochloride monoester (4), a commercially available product can be used as is, or one produced by a method known per se or a method analogous thereto can also be used.

Compound (1) obtained by the aforementioned method can be converted to a salt of compound (1) by a reaction with alkali metal hydroxide such as sodium hydroxide, potassium hydroxide and the like, alkali earth metal hydroxide such as calcium hydroxide and the like, organic amine base, or the like.

The content of component (A): compound (1) or a salt thereof in the composition of the present invention is generally 0.005 - 20 wt%, preferably 0.01 - 2 wt%, more preferably 0.1 - 2.0 wt%, relative to the total amount of the composition.

### 2. Component (B): water-soluble moisturizing component

The "water-soluble moisturizing component" in the present specification means a component having a property of easily retaining water, which results in a property of increasing water content of stratum corneum over a long period of time.

Specific examples of the "water-soluble moisturizing component" include polyols (e.g., glycerol, diglycerol, propylene glycol, 1,3-propanediol, butylene glycol, pentylene glycol, 1,5-pentanediol, dipropylene glycol, hexylene glycol, 1,2-hexanediol, 1,6-hexanediol, neopentyl glycol, isoprene glycol, low-polymerization polyethylene glycol etc.), denaturation glycol (e.g., ethylhexylglycerol, caprylylglycol etc.), saccharides (e.g., sorbitol, erythritol, mannitol, maltitol, glucose, saccharose, trehalose, tremella fuciformis polysaccharide etc.), pyrrolidone acid (PCA) and a salt thereof, betaine, caproyl proline and a salt thereof, dilauroylglutamyl lysine sodium:, peptide (polysodium aspartate, hydrolyzed protein etc.), lactic acid and a salt thereof, urea, hyaluronic acid and a derivative thereof and the like.

The "water-soluble moisturizing component" is preferably polyol or saccharide, more preferably polyol.

Particularly, as polyol, glycerol, butylene glycol or 1,3-propanediol is preferable, and glycerol or butylene glycol is more preferable.

As saccharides, sorbitol, erythritol or trehalose is preferable, and sorbitol is more preferable.

The content of component (B): water-soluble moisturizing component in the composition of the present invention is generally 0.1 - 60 wt%, preferably 0.1 - 30 wt%, relative to the total amount of the, composition.

### 3. Component (C): water

The content of component (C): water in the composition of the present invention is generally 2.0 - 95 wt%, preferably 3 - 90 wt%, more preferably 5 - 80 wt%, relative to the total amount of the composition.

### 4. Component (D): nonionic surfactant

Specific examples of the "nonionic surfactant" in the present specification include polyoxyalkylene ethers (e.g., polyoxyalkylene alkyl ether, polyoxyethylene·polyoxypropylene alkyl ether, polyoxyethylene behenyl ether, polyoxyalkylene phenyl ether, polyoxyethylene phytosterol, polyoxyethylene cholesterol, polyoxyalkylene pentaerythritol ether, polyoxyalkylene trimerol propaneether etc.), polyhydric alcohol fatty acid esters(ethylene glycol fatty acid ester, (poly)glycerol fatty acid ester (glyceryl stearate etc.), propylene glycol ester of fatty acid, sorbitan ester of fatty acid, sucrose ester of fatty acid, methylglucoside fatty acid ester etc.), polyoxyalkylene esters(polyoxyalkylene fatty acid ester, polyoxyalkylene sorbitan ester of fatty acid, polyoxyalkylene sorbitol fatty acid ester, polyoxyalkylene hydrogenated castor oil, polyoxyethylene hydrogenated castor oil fatty acid ester, polyoxyethylene alkyl ether fatty acid ester etc.), polyoxyethylene dimer diol ether, polyoxyethylene glyceryl ether fatty acid ester, ether modified dimethicones, alkyl polyglucoside, fatty acid alkanolamide, alkylamineoxide, hydrogenated lecithin and the like. Of these, polyoxyalkylene ethers, polyhydric alcohol fatty acid esters, polyoxyalkylene esters, polyoxyethylene dimer diol ether, polyoxyethylene glyceryl ether fatty acid ester and ether modified dimethicone are preferable.

The content of component (D): nonionic surfactant in the composition of the present invention is generally 0.01 - 15 wt%, preferably 0.01 - 8 wt%, more preferably 0.01 - 5 wt%, particularly preferably 0.1 - 5 wt%, relative to the total amount of the composition.

### 5. Component (E): oil agent

Specific examples of the "oil agent" in the present specification include solid or semi-solid oil (e.g., petrolatum, lanolin, ceresin, microcrystalline wax, Carnauba wax, candelilla wax, Shea butter etc.), higher fatty acid (e.g., stearic acid, isostearic acid, polyhydroxy stearic acid etc.), higher alcohol (e.g., cetyl alcohol, stearyl alcohol, behenyl alcohol, octyldodecanol, oleyl alcohol etc.), hydrocarbon oil (e.g., squalane, liquid paraffin (mineral oil), hydrogenated polyisobutene, polybutene, isododecane etc.), natural or synthetic ester oil (e.g., jojoba seed oil, isononyl isononanoate, isostearyl neopentanoate, cetyl 2-ethylhexanoate, ethylhexyl palmitate, isopropyl myristate, isopropyl palmitate, cetyl palmitate, diisostearyl malate, isocetyl isostearate, isostearyl neopentanoate, ethylhexyl methoxycinnamate, octyldodecyl/phytosteryl/behenyl lauroyl glutamate, macadamia nut oil fatty acid phytosteryl, dicaprylyl carbonate, diisopropyl sebacate, alkyl benzoate etc.), diglyceride, natural or synthetic triglycerides (e.g., corn oil, olive oil, sunflower oil, coconut oil, sweet almond oil, meadowfoam oil, grape seed oil, tri(carpylic/capric acid)glyceryl, triethylhexanoin etc.), high viscosity oil (e.g., phytosteryl/isostearyl/cetyl/stearyl/behenyl dimer dilinolate, pentaerythrityl tetraisostearate etc.), silicone oil (e.g., dimethicone, methicone, cyclomethicone, phenyltrimethicone etc.), fluorine type oil agent (e.g., perfluoropolyether, perfluorodecalin, perfluorooctane etc.) and the like. Of these, petrolatum, lanolin, stearic acid, stearyl alcohol, behenyl alcohol, squalane, liquid paraffin (mineral oil), jojoba seed oil, olive oil, triethylhexanoin, tri(carpylic/capric acid)glyceryl, dimethicone and cyclomethicone are preferable.

The oil agent may be used alone or two or more kinds thereof may be used in a mixture.

The content of component (E): oil agent in the composition of the present invention is generally 0.1 - 95 wt%, preferably 0.1 - 80 wt%, relative to the total amount of the composition.

### 6. Component (F): water-soluble polymer

The "water-soluble polymer" in the present specification means a polymer having an action to markedly increase viscosity of an aqueous solution when dissolved in water, thus affording effects of improving easy use of cosmetics, and stabilizing emulsion and dispersion. Specific examples of the "water-soluble polymer" include natural polysaccharides and a derivative thereof (e.g., xanthan gum, guar gum, locust bean gum, gellan gum, tamarind gum, quince seed gum, dextrin, carrageenan, agar, alginic acid, pectin, mannan etc.), cellulose derivative(cellulose gum, ethylcellulose, hydroxyethylcellulose, hydroxypropylcellulose, hydroxypropylmethylcellulose, carboxymethylcellulose etc.), starch derivative (e.g., α-glucan, starch glycolic acid, hydroxypropyl starch phosphoric acid etc.), synthesis nonionic polymer (e.g., high-polymerization polyethylene glycol, polyvinyl alcohol, polyvinylpyrrolidone, high-polymerization silicone etc.), gelatin and the like. Of these, xanthan gum, guar gum, hydroxyethylcellulose, hydroxypropylmethylcellulose, hydroxypropyl starch phosphoric acid, high-polymerization polyethylene glycol and polyvinylpyrrolidone are preferable.

The content of component (F): water-soluble polymer in the composition of the present invention is generally 0.01 - 10 wt%, preferably 0.01 - 5 wt%, more preferably 0.05 - 5 wt%, relative to the total amount of the composition.

The present invention also relates to a cosmetic containing the aforementioned composition of the present invention.

Specific examples of the cosmetics include basic cosmetic (e.g., skin lotion, milky lotion, makeup base, serum, night cream, facial mask, makeup remover product (cleansing gel etc.), nail cream etc.), sun care product (e.g., sunscreen, lotion for sunburn skin etc.), hair treatment agent (e.g., hair treatment, out-bath treatment, serum for hair, split end mender etc.), hair styling products (e.g., brushing lotion, curler lotion, pomado, stick pomade, hair spray for styling, hair mist, hair liquid, styling foam, hair gel, water grease etc.), shaving product (e.g., shaving cream, after-shave lotion etc.), makeup cosmetic (e.g., foundation(solid, cream, liquid etc.), BB cream, CC cream, concealer, rouge, lip gloss, eye shadow, eyeliner, blush, mascara, bronzer etc.), perfumes, lip cream, adiaphoretic, oral cosmetic, tooth paste, bath cosmetic (e.g., bathing powder, bath salt etc.) and the like.

The cosmetics of the present invention may contain components that can be generally added to a cosmetic, as long as the effect of the present invention is not inhibited. Specific examples include amino acids, amino acid derivative, chelating agent, cosmetic powder, lower alcohol, animal and plant extract, nucleic acid, vitamin, enzyme, anti-inflammatory agent, antimicrobial agent, preservative, antioxidant, ultraviolet absorber, adiaphoretic, pigment, dye, oxidation dye, pH adjuster, pearly sheen agent, wetting agent and the like.

The composition of the present invention, and a cosmetic containing the composition can be produced according to a conventional method.

### [Examples]

The present invention is concretely explained in the following by referring to Production Example and Examples. The present invention is not limited by the following Production Example and Examples. Unless particularly indicated, "%" means "wt%".

### Production Example 1: Synthesis of bis(N^{ε}-lauroyl-L-lysine)sebacoylamide disodium salt

N^{ε}-lauroyl-L-lysine (8.2 g, 25 mmol) was dissolved in water (70 g) and 25% aqueous sodium hydroxide solution (10 g), and diethyl ether (80 g) was added. Sebacoyl chloride (3.3 g, 14 mmol) was slowly added to the ether layer. The two-layer solution was stirred for about 1 hr while maintaining at 0°C, and then at room temperature for 23 hr. Then, 75% sulfuric acid was added dropwise to adjust to pH 2, the obtained white precipitate was collected by filtration, washed well with water and dried. The obtained compound was dissolved in an aqueous sodium hydroxide solution to give a 10% aqueous bis(N^{ε}-lauroyl-L-lysine)sebacoyl amide disodium salt solution.

### Production Example 2: Synthesis of bis(N^{ε}-octanoyl-L-lysine)sebacoylamide disodium salt

N^{ε}-octanoyl-L-lysine (6.8 g, 25 mmol) was dissolved in water (70 g) and 25% aqueous sodium hydroxide solution (10 g), and diethyl ether (80 g) was added. Sebacoyl chloride (3.3 g, 14 mmol) was slowly added to the ether layer. The two-layer solution was stirred for about 1 hr while maintaining at 0°C, and then at room temperature for 23 hr. Then, 75% sulfuric acid was added dropwise to adjust to pH 2, the obtained white precipitate was collected by filtration, washed well with water and dried. The obtained compound was dissolved in an aqueous sodium hydroxide solution to give a 10% aqueous bis(N^{ε}-octanoyl-L-lysine)sebacoyl amide disodium salt solution.

1H-NMR of bis(N^{ε}-octanoyl-L-lysine)sebacoyl amide (free form)

1H-NMR (400 MHz, DMSO-d6, TMS, 25°C):50.85 (t, J = 6.8 Hz, 6H), 1.20-1.29 (m, 28H), 1.32-1.38 (m, 4H), 1.45-1.50 (m, 8H), 1.54-1.59 (m, 4H), 2.02 (t, J = 7.4 Hz, 4H), 2.09 (t, J = 7.4 Hz, 4H), 2.99 (q, J = 6.5 Hz, 4H), 4.08-4.47 (m, 2H), 7.73 (t, J = 5.6 Hz, 2H), 7.97 (d, J = 8.0 Hz, 2H).

### [Example 1] Production of milky lotion

Dimethicone (90 g), olive oil (90 g), triethylhexanoin (90 g), mineral oil (70 cps) (90 g), and jojoba seed oil (300 g) were mixed. Separately, glycerol (180 g) and the compound (240 g; as 10% aqueous solution) of Production Example 1 were mixed by heating at 60°C and, when the mixture became uniform, the mixed oil mentioned above was added. The mixture was stirred at 60°C, and cooled to room temperature to give an oil gel. Separately, xanthan gum (6 g) was gradually added to purified water (1785 g) with stirring to allow for swelling. The oil gel mentioned above was dispersed therein, 3 wt% aqueous citric acid solution (129 g) was added and the mixture was sufficiently stirred to give a milky lotion (3000 g).

The obtained milky lotion contained small emulsion drops, the emulsion state thereof was good, and the milky lotion maintained smooth appearance even after lapse of 6 months.

### [Example 2] Production of transparent cleansing gel

The compound (400 g; as 10% aqueous solution) of Production Example 2 and glycerol (600 g) were mixed by stirring at 70°C. Mineral oil (1000 g) was gradually added dropwise thereto with stirring, and the mixture was stirred at 70°C and cooled to room temperature to give a transparent cleansing gel (2000 g).

### [Examples 3 - 8] [Comparative Examples 1, 2] Production and evaluation of moisturizing serum

Moisturizing sera (cosmetic composition) having compositions (unit: wt%) shown in the following Table 1 were produced, and the preparations were evaluated for smoothness, affinity to the skin, feeling of remaining on the skin, moist feeling of the skin immediately after application and 3 hr after application, and absence of stickiness of the skin immediately after application. Furthermore, the moisturizing effect of the moisturizing serum of Example 3 and the moisturizing serum of Comparative Example 1 was evaluated.

### 1. Production of moisturizing serum

Component (A) or N^{ε}-lauroyllysine, component (B), water (component (C)) and methylparaben shown in the following Table 1 were mixed and stirred at 70°C, component (D) and component (E) similarly mixed by heating at 70°C were gradually added and emulsified, and the mixture was cooled to room temperature with stirring to give a moisturizing serum. In the Table, PCA-Na: sodium pyrrolidone carboxylate, Glu: glutamic acid, Thr: threonine, Ser: serine, Pro: proline, Gly: glycine and Ala: alanine.

As shown in Fig. 1, the moisturizing serum of Example 3 showed good emulsion state with small emulsion drops, whereas the moisturizing serum of the Comparative Example 2 was observed to contain aggregation of N^{ε}-lauroyllysine powder which degrades smoothness of the preparation.

### 2. Evaluation of preparation for smoothness, affinity to the skin, feeling of remaining on the skin, moist feeling of the skin immediately after application and 3 hr after application, and absence of stickiness of the skin immediately after application

Five panelists made evaluation according to the following criteria, and the points were averaged and each item was evaluated according to the following evaluation criteria.

### (smoothness of preparation)

Appearance of cosmetic before taking with fingers, and when cosmetic of the size of one-yen coin is spread a little on the palm of hand,
5 points: uniform appearance; easy to spread, very smooth coated film
4 points: uniform appearance; rather difficult to spread, smooth coated film
3 points: uniform appearance; rather difficult to spread, coated film somewhat lacking smoothness
2 points: uniform appearance, difficult to spread, coated film clearly lacking smoothness
1 point: separation and multiple layers observed, or clearly nonuniform appearance

### (affinity to the skin)

When cosmetic of the size of *adzuki* bean is applied and spread on the back of hand,
5 points: affinity to the skin is very good
4 points: affinity to the skin if good
3 points: normal
2 points: bad affinity to the skin
1 point: very bad affinity to the skin

### (feeling of remaining on the skin)

When cosmetic of the size of *adzuki* bean is applied and spread on the back of hand,
5 points: cosmetic is felt firmly on the skin after application
4 points: cosmetic is felt on the skin after application
3 points: felt normal
2 points: less feeling of cosmetic on the skin after application
1 point: no feeling of cosmetic on the skin after application

### (moist feeling of the skin, immediately after application and 3 hr after application)

5 points: highly moist feeling
4 points: moist feeling
3 points: normal
2 points: less moist feeling
1 point: no moist feeling

### (absence of stickiness of the skin immediately after application)

5 points: completely no stickiness
4 points: mostly no stickiness
3 points: normal
2 points: rather sticky
1 point: strong stickiness

### (evaluation criteria)

⊙: average not less than 4.0
○: average not less than 3.0 and less than 4.0
Δ: average not less than 2.0 and less than 3.0
×: average less than 2.0

The results are shown in Table 1.

**Table 1**

| | | Ex. 3 | Ex. 4 | Ex. 5 | Ex. 6 | Ex. 7 | Ex. 8 | Comp. Ex. 1 | Comp. Ex. 2 |
|---|---|---|---|---|---|---|---|---|---|
| component (A) | compound of Production Example 1 (as 10% aqueous solution) | 3.00 | 2.00 | 1.00 | 0.50 | 0.20 | | | |
| | compound of Production Example 2 (as 10% aqueous solution) | | | | | | 3.00 | | |
| | N^{ε}-lauroyllysine | | | | | | | | 0.30 |
| component (B) | butylene glycol | 7.00 | 7.00 | 7.00 | 7.00 | 7.00 | 7.00 | 7.00 | 7.00 |
| | sorbitol | 0.21 | 0.21 | 0.21 | 0.21 | 0.21 | 0.21 | 0.21 | 0.21 |
| | betaine | 1.50 | 1.50 | 1.50 | 1.50 | 1.50 | 1.50 | 1.50 | 1.50 |
| | PCA-Na | 0.50 | 0.50 | 0.50 | 0.50 | 0.50 | 0.50 | 0.50 | 0.50 |
| component (C) | water | 68.98 | 69.93 | 70.88 | 71.35 | 71.64 | 68.98 | 71.88 | 71.58 |
| component (D) | polyoxyethylene(20)behenyl ether | 1.60 | 1.60 | 1.60 | 1.60 | 1.60 | 1.60 | 1.60 | 1.60 |
| | polyoxyethylene(5)behenyl ether | 0.10 | 0.15 | 0.20 | 0.23 | 0.24 | 0.10 | 0.20 | 0.20 |
| | glyceryl stearate | 0.20 | 0.20 | 0.20 | 0.20 | 0.20 | 0.20 | 0.20 | 0.20 |
| | hydrogenated lecithin | 1.00 | 1.00 | 1.00 | 1.00 | 1.00 | 1.00 | 1.00 | 1.00 |
| component (E) | cetyl alcohol | 0.50 | 0.50 | 0.50 | 0.50 | 0.50 | 0.50 | 0.50 | 0.50 |
| | stearyl alcohol | 1.00 | 1.00 | 1.00 | 1.00 | 1.00 | 1.00 | 1.00 | 1.00 |
| | squalane | 7.00 | 7.00 | 7.00 | 7.00 | 7.00 | 7.00 | 7.00 | 7.00 |
| | isocetyl isostearate | 5.00 | 5.00 | 5.00 | 5.00 | 5.00 | 5.00 | 5.00 | 5.00 |
| | cetyle palmitate | 0.50 | 0.50 | 0.50 | 0.50 | 0.50 | 0.50 | 0.50 | 0.50 |
| | octyldodecyl/phytosteryl/behenyl lauroyl glutamate | 1.50 | 1.50 | 1.50 | 1.50 | 1.50 | 1.50 | 1.50 | 1.50 |
| others | methylparaben | 0.20 | 0.20 | 0.20 | 0.20 | 0.20 | 0.20 | 0.20 | 0.20 |
| | Glu | 0.04 | 0.04 | 0.04 | 0.04 | 0.04 | 0.04 | 0.04 | 0.04 |
| | Thr | 0.01 | 0.01 | 0.01 | 0.01 | 0.01 | 0.01 | 0.01 | 0.01 |
| | Ser | 0.08 | 0.08 | 0.08 | 0.08 | 0.08 | 0.08 | 0.08 | 0.08 |
| | Pro | 0.01 | 0.01 | 0.01 | 0.01 | 0.01 | 0.01 | 0.01 | 0.01 |
| | Gly | 0.05 | 0.05 | 0.05 | 0.05 | 0.05 | 0.05 | 0.05 | 0,05 |
| | Ala | 0.02 | 0.02 | 0.02 | 0.02 | 0.02 | 0.02 | 0.02 | 0.02 |
| | total (wt%) | 100.00 | 100.00 | 100.00 | 100.00 | 100.00 | 100.00 | 100.00 | 100.00 |
| evaluation | smoothness of preparation, next day of preparation | ⊙ | ⊙ | ⊙ | ⊙ | ⊙ | ⊙ | ⊙ | Δ |
| | smoothness of preparation, after 1 month at 40°C | ○ | ○ | ⊙ | ⊙ | ⊙ | ⊙ | ○ | × |
| | affinity to the skin | ⊙ | ⊙ | ⊙ | ⊙ | ⊙ | ⊙ | Δ | ⊙ |
| | feeling of remaining on the skin | ⊙ | ⊙ | ⊙ | ⊙ | ⊙ | ⊙ | × | Δ |
| | moist feeling of the skin immediately after application | ○ | ⊙ | ⊙ | ⊙ | ○ | ⊙ | Δ | ○ |
| | absence of stickiness of the skin immediately after application | ⊙ | ⊙ | ⊙ | ⊙ | ⊙ | ⊙ | × | ⊙ |
| | moist feeling of the skin, 3 hr after application | ⊙ | ⊙ | ⊙ | ○ | ○ | ⊙ | × | ○ |

The moisturizing sera of the present invention were confirmed to show superior effects in all evaluation items.

### 3. Evaluation of moisturizing effect

A consecutive use test wherein 6 test subjects are applied with the moisturizing serum of Example 3 and the moisturizing serum of Comparative Example 1 each onto the inner side of the left and right lower arms, morning and evening, was performed for 3 weeks, and the water content of the stratum corneum at the application site before and after the consecutive use test was measured as skin capacitance value by using Corneometer CM 825 manufactured by Courage + Khazaka. The measurement after the consecutive use test was performed at least 3 hr after the final application. The skin capacitance value at the site of application with the moisturizing serum of Comparative Example 1 increased by 3.8 on average, whereas the skin capacitance value at the site of application with the moisturizing serum of Example 3 increased by 9.8 on average, and it was confirmed that the moisturizing serum of Example 3 showed a superior moisturizing effect.

### [Example 9] [Comparative Examples 3, 4] Production and evaluation of lotion for sunburn skin

Lotion for sunburn skin (cosmetic composition) having compositions (unit: wt%) shown in the following Table 2 were produced, and the preparations were evaluated for smoothness, affinity to the skin, feeling of remaining on the skin, moist feeling of the skin immediately after application and 3 hr after application, and absence of stickiness of the skin immediately after application. Furthermore, the lotion for sunburn skin of Example 9 and the lotion for sunburn skin of Comparative Example 3 were evaluated for cool feeling after application.

### 1. Production of lotion for sunburn skin

Ethanol, d-camphor and menthyl ethyl amino oxalic acid were mixed at room temperature (mixture A). Separately, all components except the above-mentioned component 3 were dissolved at 70°C, and cooled to 40°C with stirring. Mixture A was added thereto and cooled to room temperature with stirring to give lotion for sunburn skin.

### 2. Evaluation of preparation for smoothness, affinity to the skin, feeling of remaining on the skin, moist feeling of the skin immediately after application and 3 hr after application, and absence of stickiness of the skin immediately after application

Each item was evaluated by a method similar to that for the aforementioned moisturizing serum.

The results are shown in Table 2.

**Table 2**

| | | Ex. 9 | Comp. Ex. 3 | Comp. Ex. 4 |
|---|---|---|---|---|
| component (A) | compound of Production Example 2 (as 10% solution) | 3.00 | | |
| | N^{ε}-octanoyllysine | | | 0.30 |
| component (B) | glycerol | 5.00 | 5.00 | 5.00 |
| | trehalose | 0.30 | 0.30 | 0.30 |
| component (C) | water | 86.95 | 89.95 | 89.65 |
| component (D) | polyoxyethylene(30)polyoxy propylene (6) decyltetradecyl ether | 1.00 | 1.00 | 1.00 |
| | polyoxyethylene(60) hydrogenated castor oil | 0.50 | 0.50 | 0.50 |
| others | ethanol | 3.00 | 3.00 | 3.00 |
| | d-camphor | 0.02 | 0.02 | 0.02 |
| | menthyl ethyl amino oxalic acid | 0.10 | 0.10 | 0.10 |
| | salicylic acid | 0.01 | 0.01 | 0.01 |
| | dipotassium glycyrrhizinate | 0.05 | 0.05 | 0.05 |
| | edetate trisodium | 0.05 | 0.05 | 0.05 |
| | phenoxyethanol | 0.20 | 0.20 | 0.20 |
| | total (wt%) | 100.00 | 100.00 | 100.00 |
| | | | | |
| evaluation | smoothness of preparation, next day of preparation | ⊙ | ⊙ | × |
| | smoothness of preparation, after 1 month at 40°C | ○ | ⊙ | × |
| | affinity to the skin | ⊙ | Δ | Δ |
| | feeling of remaining on the skin | ⊙ | Δ | Δ |
| | moist feeling of the skin immediately after application | ○ | ○ | ○ |
| | absence of stickiness of the skin immediately after application | ○ | Δ | ⊙ |
| | moist feeling of the skin, 3 hr after application | ⊙ | × | Δ |

The lotion for sunburn skin of the present invention was confirmed to show superior effects in all evaluation items.

### 3. Evaluation of cool feeling after application

The lotion for sunburn skin of Example 9 and the lotion for sunburn skin of Comparative Example 3 (each 20 µL) was each placed on the back of left hand and spread thereon. While the cool feeling immediately after application was of the same level for the both, a cool feeling was felt even 30 min after application at the site on which the skin lotion of Example 9 was applied, and it was confirmed that the lotion for sunburn skin of Example 9 is superior in the retention of the effect.

### [Industrial Applicability]

The present invention can provide a stable composition, which is superior in the affinity and adhesion to the skin and hair, easily provides effects as a cosmetic such as moisturizing effect and conditioning effect and the like, and is also superior in the sustainability of the effect.

## Claims

1. A cosmetic composition comprising component (A): a compound represented by the formula (1) 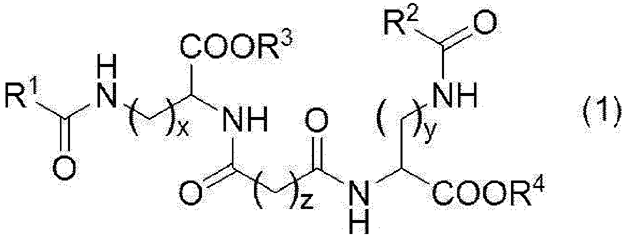 wherein
R¹ and R² are each independently an alkyl group having 5 - 21 carbon atoms or an alkenyl group having 5 - 21 carbon atoms,
R³ and R⁴ are each independently a hydrogen atom, an alkyl group having 1 - 22 carbon atoms or an alkenyl group having 2 - 22 carbon atoms,
z is an integer of not less than 0,
x and y are each independently an integer of 2 - 4, or a salt thereof,
component (B): a water-soluble moisturizing component selected from a polyol, denaturation glycol, a saccharide, pyrrolidone acid (PCA) or a salt thereof, betaine, caproyl proline or a salt thereof, dilauroylglutamyl lysine sodium, a peptide, lactic acid or a salt thereof, urea, hyaluronic acid or a derivative thereof; and
component (C): water;
wherein the amount of component (A) is between 0.005 - 20 wt.%, the amount of component (B) is between 0.1-60 wt.%, and the content of component (C) is between 2.0 to 95 wt%, the weight percentages being relative to the total amount of the composition.

2. The cosmetic composition according to claim 1, wherein component (A) is a compound of the aforementioned formula (1) wherein z is an integer of 0 - 10, or a salt thereof.

3. The cosmetic composition according to claim 1 or 2, wherein component (A) is a compound of the aforementioned formula (1) wherein z is 7 or 8, or a salt thereof.

4. The cosmetic composition according to any one of claims 1 to 3, wherein component (A) is a compound of the aforementioned formula (1) wherein x and y are each 4, or a salt thereof.

5. The cosmetic composition according to any one of claims 1 to 4, wherein component (A) is a compound of the aforementioned formula (1) wherein R¹ and R² are each independently a straight-chain alkyl group having 5 - 15 carbon atoms, or a salt thereof.

6. The cosmetic composition according to any one of claims 1 to 5, wherein component (A) is a compound of the aforementioned formula (1) wherein R³ and R⁴ are each a hydrogen atom, or a salt thereof.

7. The cosmetic composition according to any one of claims 1 to 5, wherein component (A) is a compound of the aforementioned formula (1) wherein R¹ and R² are each independently a straight-chain alkyl group having 5 - 15 carbon atoms, R³ and R⁴ are each a hydrogen atom, z is an integer of 0 - 10, and x and y are each 4, or a salt thereof.

8. The cosmetic composition according to any one of claims 1 to 5, wherein component (A) is a compound of the aforementioned formula (1) wherein R¹ and R² are each a straight-chain alkyl group having 5 - 15 carbon atoms, R³ and R⁴ are each a hydrogen atom, z is 7 or 8, and x and y are each 4, or a salt thereof.

9. The cosmetic composition according to any one of claims 1 to 8, wherein component (A) is a compound selected from bis(N^{ε}-lauroyl-L-lysine)sebacoyl amide or a salt thereof, and bis(N^{ε}-octanoyl-L-lysine)sebacoyl amide, or a salt thereof.

10. The cosmetic composition according to any one of claims 1 to 9, wherein component (B) is at least one kind of water-soluble moisturizing component selected from the group consisting of polyols and saccharides.

11. The cosmetic composition according to any one of claims 1 to 10, further comprising component (D): a nonionic surfactant.

12. The cosmetic composition according to claim 11, wherein component (D) is at least one kind of nonionic surfactant selected from the group consisting of polyoxyalkylene ethers, polyhydric alcohol fatty acid esters, polyoxyalkylene esters, polyoxyethylene dimer diol ether, polyoxyethylene glyceryl ether fatty acid ester and ether modified dimethicone.

13. The cosmetic composition according to any one of claims 1 to 12, further comprising component (E): an oil agent and/or component (F): a water-soluble polymer.

14. Use of the composition according to any one of claims 1 to 13 for skin care or hair care.

## Patentansprüche

1. Kosmetische Zusammensetzung, umfassend eine Komponente (A): eine Verbindung der Formel (1) 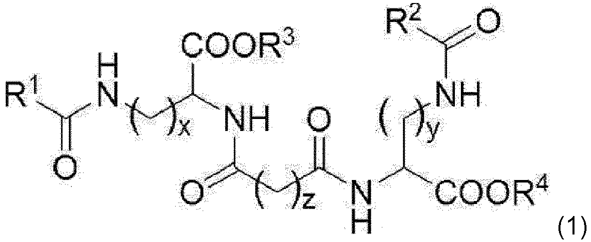 wobei
R¹ und R² jeweils unabhängig voneinander eine Alkylgruppe mit 5-21 Kohlenstoffatomen oder eine Alkenylgruppe mit 5-21 Kohlenstoffatomen sind,
R³ und R⁴ jeweils unabhängig voneinander ein Wasserstoffatom, eine Alkylgruppe mit 1-22 Kohlenstoffatomen oder eine Alkenylgruppe mit 2-22 Kohlenstoffatomen sind,
z eine ganze Zahl von nicht weniger als 0 ist,
x und y jeweils unabhängig voneinander eine ganze Zahl von 2-4 sind, oder ein Salz davon,
Komponente (B): eine wasserlösliche feuchtigkeitsspendende Komponente, ausgewählt unter einem Polyol, Denaturierungsglykol, Saccharid, Pyrrolidonsäure (PCA) oder einem Salz davon, Betain, Caproylprolin oder einem Salz davon, Dilauroylglutamyllysin-Natrium, einem Peptid, Milchsäure oder einem Salz davon, Harnstoff, Hyaluronsäure oder einem Derivat davon; und
Komponente (C): Wasser;
wobei die Menge der Komponente (A) zwischen 0.005 - 20 Gew.-% beträgt, die Menge der Komponente (B) zwischen 0,1-60 Gew.-% und der Gehalt der Komponente (C) zwischen 2,0 bis 95 Gew.-% beträgt, wobei die Gewichtsprozentsätze auf die Gesamtmenge der Zusammensetzung bezogen sind.

2. Kosmetische Zusammensetzung nach Anspruch 1, worin die Komponente (A) eine Verbindung der vorgenannten Formel (1), worin z eine ganze Zahl von 0-10 ist, oder ein Salz davon ist.

3. Kosmetische Zusammensetzung nach Anspruch 1 oder 2, worin die Komponente (A) eine Verbindung der vorgenannten Formel (1), worin z 7 oder 8 ist, oder ein Salz davon ist.

4. Kosmetische Zusammensetzung nach einem der Ansprüche 1 bis 3, wobei die Komponente (A) eine Verbindung der vorgenannten Formel (1), worin x und y jeweils 4 sind, oder ein Salz davon ist.

5. Kosmetische Zusammensetzung nach einem der Ansprüche 1 bis 4, worin die Komponente (A) eine Verbindung der vorgenannten Formel (1), worin R¹ und R² jeweils unabhängig voneinander eine geradkettige Alkylgruppe mit 5-15 Kohlenstoffatomen sind, oder ein Salz davon ist.

6. Kosmetische Zusammensetzung nach einem der Ansprüche 1 bis 5, worin die Komponente (A) eine Verbindung der vorgenannten Formel (1), worin R³ und R⁴ jeweils ein Wasserstoffatom sind, oder ein Salz davon ist.

7. Kosmetische Zusammensetzung nach einem der Ansprüche 1 bis 5, worin die Komponente (A) eine Verbindung der vorgenannten Formel (1), worin R¹ und R² jeweils unabhängig voneinander eine geradkettige Alkylgruppe mit 5-15 Kohlenstoffatomen sind, R³ und R⁴ jeweils ein Wasserstoffatom sind, z eine ganze Zahl von 0-10 ist und x und y jeweils 4 sind, oder ein Salz davon ist.

8. Kosmetische Zusammensetzung nach einem der Ansprüche 1 bis 5, worin die Komponente (A) eine Verbindung der vorstehend genannten Formel (1), worin R¹ und R² jeweils eine geradkettige Alkylgruppe mit 5-15 Kohlenstoffatomen sind, R³ und R⁴ jeweils ein Wasserstoffatom sind, z 7 oder 8 ist und x und y jeweils 4 sind, oder ein Salz davon ist.

9. Kosmetische Zusammensetzung nach einem der Ansprüche 1 bis 8, worin die Komponente (A) eine Verbindung ist, die aus Bis(N^{ε}-lauroyl-L-lysin)sebacoylamid oder einem Salz davon und Bis(N^{ε}-octanoyl-L-lysin)sebacoylamid oder einem Salz davon ausgewählt ist.

10. Kosmetische Zusammensetzung nach einem der Ansprüche 1 bis 9, worin Komponente (B) mindestens eine Art wasserlösliche feuchtigkeitsspendende Komponente ist, ausgewählt aus der aus Polyolen und Sacchariden bestehenden Gruppe.

11. Kosmetische Zusammensetzung nach einem der Ansprüche 1 bis 10, ferner enthaltend die Komponente (D): ein nichtionisches Tensid.

12. Kosmetische Zusammensetzung nach Anspruch 11, worin die Komponente (D) mindestens eine Art nichtionisches Tensid ist, ausgewählt aus der Gruppe bestehend aus Polyoxyalkylenethern, mehrwertigen Alkohol-Fettsäureestern, Polyoxyalkylenestern, Polyoxyethylendimerdiolether, Polyoxyethylenglycerylether-Fettsäureester und ethermodifiziertem Dimethicon.

13. Kosmetische Zusammensetzung nach einem der Ansprüche 1 bis 12, ferner umfassend die Komponente (E): ein Ölmittel und/oder die Komponente (F): ein wasserlösliches Polymer.

14. Verwendung der Zusammensetzung nach einem der Ansprüche 1 bis 13 zur Haut- oder Haarpflege.

## Revendications

1. Composition cosmétique comprenant un composant (A) : un composé représenté par la formule (1) 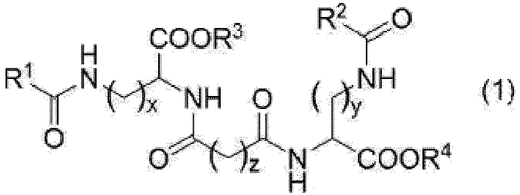 dans laquelle :
R¹ et R² sont chacun, de manière indépendante, un groupe alkyle qui comporte de 5 à 21 atomes de carbone ou un groupe alkényle qui comporte de 5 à 21 atomes de carbone ;
R³ et R⁴ sont chacun, de manière indépendante, un atome d'hydrogène, un groupe alkyle qui comporte de 1 à 22 atome(s) de carbone ou un groupe alkényle qui comporte de 2 à 22 atomes de carbone ;
z est un entier non inférieur à 0 ;
x et y sont chacun, de manière indépendante, un entier de 2 à 4, ou un sel de celui-ci ;
un composant (B) : un composant hydratant soluble dans l'eau sélectionné parmi un polyol, un glycol de dénaturation, un saccharide, l'acide pyrrolidone (PCA) ou un sel de celui-ci , la bétaïne, la caproyl proline ou un sel de celle-ci , la sodium dilauroylglutamyl lysine, un peptide, l'acide lactique ou un sel ade celui-ci, l'urée, l'acide hyaluronique ou un dérivé de celui-ci ; et
un composant (C) : de l'eau ;
dans laquelle la quantité du composant (A) se situe entre 0,005 et 20 % en poids, la quantité du composant (B) se situe entre 0,1 et 60 % en poids et la quantité du composant (C) se situe entre 2,0 et 95 % en poids, les pourcentages en poids étant rapportés à la quantité totale de la composition.

2. Composition cosmétique selon la revendication 1, dans laquelle le composant (A) est un composé de la formule (1) mentionnée ci-avant dans laquelle z est un entier de 0 à 10, ou un sel de celui-ci .

3. Composition cosmétique selon la revendication 1 ou 2, dans laquelle le composant (A) est un composé de la formule (1) mentionnée ci-avant dans laquelle z est 7 ou 8, ou un sel de celui-ci .

4. Composition cosmétique selon l'une quelconque des revendications 1 à 3, dans laquelle le composant (A) est un composé de la formule (1) mentionnée ci-avant dans laquelle x et y sont chacun 4, ou un sel de celui-ci .

5. Composition cosmétique selon l'une quelconque des revendications 1 à 4, dans laquelle le composant (A) est un composé de la formule (1) mentionnée ci-avant dans laquelle R¹ et R² sont chacun, de manière indépendante, un groupe alkyle à chaîne linéaire qui comporte de 5 à 15 atomes de carbone, ou un sel de celui-ci.

6. Composition cosmétique selon l'une quelconque des revendications 1 à 5, dans laquelle le composant (A) est un composé de la formule (1) mentionnée ci-avant dans laquelle R³ et R⁴ sont chacun un atome d'hydrogène, ou un sel de celui-ci.

7. Composition cosmétique selon l'une quelconque des revendications 1 à 5, dans laquelle le composant (A) est un composé de la formule (1) mentionnée ci-avant dans laquelle R¹ et R² sont chacun, de manière indépendante, un groupe alkyle à chaîne linéaire qui comporte de 5 à 15 atomes de carbone, R³ et R⁴ sont chacun un atome d'hydrogène, z est un entier de 0 à 10 et x et y sont chacun 4, ou un sel de celui-ci.

8. Composition cosmétique selon l'une quelconque des revendications 1 à 5, dans laquelle le composant (A) est un composé de la formule (1) mentionnée ci-avant dans laquelle R¹ et R² sont chacun un groupe alkyle à chaîne linéaire qui comporte de 5 à 15 atomes de carbone, R³ et R⁴ sont chacun un atome d'hydrogène, z est 7 ou 8 et x et y sont chacun 4, ou un sel de celui-ci.

9. Composition cosmétique selon l'une quelconque des revendications 1 à 8, dans laquelle le composant (A) est un composé qui est sélectionné parmi l'amide de bis(N^{ε}-lauroyl-L-lysine)sébacoyle ou un sel de celui-ci et l'amide de bis (N^{ε}-octanoyl-L-lysine) sébacoyle ou un sel de celui-ci.

10. Composition cosmétique selon l'une quelconque des revendications 1 à 9, dans laquelle le composant (B) est au moins un type de composant hydratant soluble dans l'eau qui est sélectionné dans le groupe constitué par les polyols et les saccharides.

11. Composition cosmétique selon l'une quelconque des revendications 1 à 10, comprenant en outre un composant (D) : un tensioactif non ionique.

12. Composition cosmétique selon la revendication 11, dans laquelle le composant (D) est au moins un type de tensioactif non ionique sélectionné dans le groupe constitué par les éthers de polyoxyalkylène, les esters d'acides gras d'alcool polyhydrique, les esters de polyoxyalkylène, l'éther d'un dimère de diol et de polyoxyéthylène, l'ester d'acides gras de glycéryl éther de polyoxyéthylène et le diméthicone modifié par éther.

13. Composition cosmétique selon l'une quelconque des revendications 1 à 12, comprenant en outre un composant (E) : un agent huileux et/ou un composant (F) : un polymère soluble dans l'eau.

14. Utilisation de la composition selon l'une quelconque des revendications 1 à 13 pour les soins de la peau ou les soins des cheveux.
